# EUROPEAN PATENT APPLICATION

(11) **EP 4 806 910 A1**
(43) Date of publication of application: **16.09.2026**
(21) Application number: 24887923.1
(22) Date of filing: 05.11.2024
(51) Int. Cl.: C07K 14/605, A61K 38/26, A61P 3/00, A61P 3/10, A61P 3/04, A61P 9/12, A61P 25/00, A61P 9/00, A61P 1/00

(54) **TRI-AGONIST COMPOUND**

(30) Priority: 06.11.2023 CN 202311462759
(71) Applicant: Chengdu Aoda Biotechnology Co., Ltd, Chengdu, Sichuan 610041 (CN)
(72) Inventor: ZHOU, Shuliang, Chengdu, Sichuan 610041 (CN); GAO, Junping, Chengdu, Sichuan 610041 (CN); WANG, Peng, Chengdu, Sichuan 610041 (CN)
(74) Representative: PGA S.p.A., Milano, Succursale di Lugano
(86) International application number: PCT/CN2024/129854
(87) International publication number: WO 2025/098311

(57) **Abstract**

The present invention belongs to the field of pharmaceutical synthesis. Provided is a GLP-1/GIP/GCG tri-agonist compound, the GLP-1/GIP/GCG tri-agonist compound being used to prepare a pharmaceutical composition for treating diseases, and a use of the pharmaceutical composition in the preparation of a medicament for treating at least one of the following diseases, the treated diseases comprising type II diabetes, impaired glucose tolerance, type I diabetes, obesity, hypertension, metabolic syndrome, dyslipidemia, cognitive impairment, atherosclerosis, myocardial infarction, coronary heart disease, cardiovascular disease, stroke, inflammatory bowel syndrome and/or dyspepsia or gastric ulcers, liver fibrosis, and pulmonary fibrosis.

## Description

This application claims the priority of Chinese Patent Application No. 202311462759.7, filed with the China National Intellectual Property Administration on November 6, 2023, and titled with "TRI-AGONIST COMPOUND", which is hereby incorporated by reference in its entirety.

### FIELD

The present disclosure relates to a GLP-1/GIP/GCG tri-agonist compound and use thereof. The compound is a modified tri-agonist of glucagon-like peptide-1 (GLP-1), human glucose-dependent insulinotropic polypeptide (GIP), and glucagon (GCG).

### BACKGROUND

The most common side effects of glucagon-like peptide-1 (GLP-1) compounds are ineffective glycemic control and weight loss. Glucose-dependent insulinotropic polypeptide (GIP) alone exhibits only very modest efficacy of lowering glucose in patients with type 2 diabetes. Native GIP and GLP-1 can both be rapidly inactivated by the ubiquitously present protease dipeptidyl peptidase IV (DPP-IV), and therefore can only be used for short-term metabolic control.

GIP is a 42-amino-acid gastrointestinal regulatory peptide, which exerts physiological functions in glucose homeostasis by stimulating insulin secretion from pancreatic β-cells in the presence of glucose and protecting pancreatic β-cells. GLP-1 is a 37-amino-acid peptide that stimulates insulin secretion, protects pancreatic β-cells, and inhibits glucagon secretion, gastric emptying, and food intake, thereby leading to weight reduction. GIP and GLP-1 are referred to as incretins; incretin receptor signaling plays a key physiological role in glucose homeostasis. Under normal physiology, GIP and GLP-1 are secreted from the intestine after meals, and these incretins enhance physiological responses to food, including satiety, insulin secretion, and nutrient disposal.

GCG, also known as glucagon, anti-insulin hormone, or insulin B, is a hormone secreted by pancreatic α-cells of vertebrate pancreas along with insulin. It antagonizes insulin and functions to increase blood glucose levels.

Recent studies indicate that GLP-1/GIP/GCG tri-receptor agonist compounds not only provide improved glycemic control, but also exhibit significant effects in reducing body weight and treating non-alcoholic fatty liver disease.

### SUMMARY

In view of this, the present disclosure provides a GLP-1/GIP/GCG tri-agonist compound and use thereof. The compound is a modified tri-agonist of glucagon-like peptide-1 (GLP-1), human glucose-dependent insulinotropic polypeptide (GIP), and glucagon (GCG).

In a first aspect, an embodiment of the present disclosure provides a GLP-1/GIP/GCG tri-agonist compound having a structure of Formula I:

Tyr-Aib-Gln-Gly-Thr-AAl-Thr-Ser-Asp-Tyr-Ser-Ile-αMeLeu-Leu-Asp-Lys-Lys((CO(C H₂)ₙ₁PEGₙ₂)ₙ₃-(AA2)ₙ₄-CO(CH₂)ₙ₅-COOH)-Ala-Gln-Ab-Ala-Phe-Ile-Glu-Tyr-Leu-Leu-Glu-(5- Ava)-Pro-Ser-Ser-Gly-Ala-Pro-Pro-Pro-Ser-AA3 Formula I

wherein:
AA1 is any one selected from the group consisting of αMePhe and αMePhe(2F);
AA2 is any one selected from the group consisting of γGlu, δAad, εApm, and ζAsu;
AA3 is any one selected from the group consisting of amino and hydroxyl;
n1=1-5, n2=1-30, n3=0 or n3=1-5, n4=0 or n4=1-5, and n5=10-30.

Optionally, for the GLP-1/GIP/GCG tri-agonist compound, AA1 is αMePhe, AA2 is γGlu, AA3 is amino, n1=1-5, n2 =1-30, n3=0 or n3 =1-5, n4=0 or n4=1-5, and n5=10-30; preferably, AA1 is αMePhe, AA2 is γGlu, AA3 is amino, n1=1, n2=2, n3=1, n4=1, and n5=18.

Optionally, for the GLP-1/GIP/GCG tri-agonist compound, AA1 is αMePhe, AA2 is δAad, AA3 is amino, n1=1-5, n2=1-30, n3=0 or n3=1-5, n4=0 or n4=1-5, and n5=10-30; preferably, AA1 is αMePhe, AA2 is δAad, AA3 is amino, n1=1, n2=2, n3=1, n4=1, and n5=18.

Optionally, for the GLP-1/GIP/GCG tri-agonist compound, AA1 is αMePhe(2F), AA2 is γGlu, AA3 is amino, n1=1-5, n2=1-30, n3=0 or n3=1-5, n4=0 or n4=1-5, and n5=10-30; preferably, AA1 is αMePhe(2F), AA2 is γGlu, AA3 is amino, n1=1, n2=2, n3=1, n4=1, and n5=18.

Optionally, for the GLP-1/GIP/GCG tri-agonist compound, AA1 is αMePhe(2F), AA2 is δAad, AA3 is amino, n1=1-5, n2=1-30, n3=0 or n3=1-5, n4=0 or n4=1-5, and n5=10-30; preferably, AA1 is αMePhe(2F), AA2 is δAad, AA3 is amino, n1=1, n2=2, n3=1, n4=1, and n5=18.

In some specific embodiments of the present disclosure, the GLP-1/GIP/GCG tri-agonist compound has:
(I) an amino acid sequence set forth in any one of SEQ ID NOs: 2 to 13;
(II) an amino acid sequence obtained by substitution, deletion, or addition of 1, 2, 3, or more amino acids to the amino acid sequence of (I) and having the same function as the amino acid sequence of (I); or
(III) an amino acid sequence having 60%, 65%, 70%, 75%, 80%, 85%, 90%, 95%, 96%, 97%, 98%, 99% or more sequence identity to the amino acid sequence of (I) or (II).

Optionally, for the GLP-1/GIP/GCG tri-agonist compound, the tri-agonist compound includes a pharmaceutically acceptable salt, chelate, or non-covalent complex formed by the compound, a precursor of the compound, or any mixture thereof.

In a second aspect, an embodiment of the present disclosure provides use of the GLP-1/GIP/GCG tri-agonist compound according to any one of the first aspect in the manufacture of a pharmaceutical composition for treating a disease.

Optionally, the pharmaceutical composition is for treating at least one disease selected from the group consisting of type II diabetes, impaired glucose tolerance, type I diabetes, obesity, hypertension, metabolic syndrome, dyslipidemia, cognitive disorder, atherosclerosis, myocardial infarction, coronary heart disease, cardiovascular disease, stroke, inflammatory bowel syndrome and/or dyspepsia or gastric ulcer, hepatic fibrosis disease, and pulmonary fibrosis disease.

Unless otherwise indicated, the numerical values used herein to represent quantities of different components and reaction conditions are to be understood, in all instances, as being modified by the terms "about" or "approximately." Accordingly, unless otherwise specifically indicated, the numerical parameters set forth in the description below and the claims are approximations that may vary depending upon the respective experimental conditions due to the different inherent standard deviations.

In the present disclosure, when there is any discrepancy or ambiguity between the chemical structural formula and the chemical name of a compound, the chemical structural formula shall prevail in defining the compound. The compounds described herein may contain one or more chiral centers and/or double bonds or similar structural features, and may therefore exist as stereoisomers, including double bond isomers (such as geometric isomers), enantiomers, or diastereomers. Accordingly, any chemical structure described herein, whether in whole or in part that contains such features, encompasses all possible enantiomers and diastereomers of the compound, including any individual stereoisomer (e.g., a single geometric isomer, a single enantiomer, or a single diastereomer) as well as any mixtures thereof. Such racemates and stereoisomeric mixtures may be further resolved into their component enantiomers or stereoisomers by those skilled in the art using various separation techniques or asymmetric synthesis methods.

The compounds of Formula I include, but not limited to, optical isomers, racemates, and/or other mixtures thereof In the above cases, individual enantiomers or diastereomers, such as optically active isomers, may be obtained by asymmetric synthesis or by resolution of racemates. Resolution of racemates may be achieved by various methods, such as conventional recrystallization with a resolving agent or chromatographic techniques. In addition, the compounds of Formula I also include cis- and/or trans-isomers associated with double bonds.

The compounds described in the present disclosure include, but not limited to, the compounds represented by Formula I and all pharmaceutically acceptable forms thereof Such pharmaceutically acceptable forms include various pharmaceutically acceptable salts, solvates, complexes, chelates, non-covalent complexes, prodrugs based on the foregoing forms, and any mixtures of the above.

In a third aspect, the present disclosure provides a drug, comprising the GLP-1/GIP/GCG tri-agonist compound.

In a fourth aspect, the present disclosure provides a pharmaceutical composition, comprising the GLP-1/GIP/GCG tri-agonist compound and any additional active ingredient.

In a fifth aspect, the present disclosure provides a method for treating a disease, comprising administering the GLP-1/GIP/GCG tri-agonist compound, the drug, or the pharmaceutical composition.

The compounds represented by Formula I provided by the disclosure are stable in properties and are highly active GLP-1/GIP/GCG tri-receptor agonist compounds, exhibiting significant effects in reducing blood glucose level and body weight.

### DETAILED DESCRIPTION

The present disclosure provides a tri-agonist compound. Those skilled in the art can, in light of the teachings herein, implement the present disclosure by appropriately modifying process parameters. It should be particularly noted that all similar substitutions and modifications would be obvious to those skilled in the art and are intended to be included within the scope of the present disclosure. The methods and uses of the present disclosure have been described with reference to preferred embodiments, and it will be obvious to those skilled in the art that, without departing from the content, spirit and scope of the present disclosure, the methods and uses described herein may be modified or appropriately varied and combined to implement and apply the present disclosure.

The present disclosure provides a GLP-1/GIP/GCG tri-receptor agonist compound and use thereof. Those skilled in the art can, in light of the teachings herein, implement the present disclosure by appropriately modifying relevant parameters. It should be particularly noted that all similar substitutions and modifications would be obvious to those skilled in the art and are intended to be included within the scope of the present disclosure. The methods and uses of the present disclosure have been described with reference to preferred embodiments, and it will be obvious to those skilled in the art that, without departing from the content, spirit and scope of the present disclosure, the methods and uses described herein may be modified or appropriately varied and combined to implement and apply the present disclosure.

The names corresponding to the abbreviations used in the present disclosure are shown in the table below:

| Abbreviation | Name | Abbreviation | N ame |
|---|---|---|---|
| Fmoc | 9-fluorenylmethoxycarbon yl | OtBu | tert-butoxy |
| tBu | tert-butyl | Boc | tert-butoxycarbonyl |
| Trt | triphenylmethyl | Pbf | (2,3-dihydro-2,2,4,6,7-pentamethyl benzofuran-5-yl)sulfonyl |
| Ala | alanine | Leu | leucine |
| Arg | arginine | Lys | lysine |
| Asn | asparagine | Phe | phenylalanine |
| Asp | aspartic acid | Pro | proline |
| Cys | cysteine | Ser | serine |
| Gln | glutamine | Thr | threonine |
| Glu | glutamic acid | Trp | tryptophan |
| Gly | glycine | Tyr | tyrosine |
| His | histidine | Val | valine |
| Ile | isoleucine | Ada | 2-aminoadipic acid |
| Aib | aminoisobutyric acid | Apm | 2-aminopimelic acid |
| 5-Ava | 5-aminovaleric acid | Asu | 2-aminosuberic acid |

### Example 1: Manufacture of compounds

A manufacture method, comprising: preparing a peptide-resin by solid-phase peptide synthesis, subjecting the peptide-resin to acidolysis to obtain a crude product, and finally purifying the crude product to obtain a purified product; wherein the step of preparing the peptide-resin by solid-phase peptide synthesis is carried out by sequentially coupling protected amino acids or protected amino acid fragments corresponding to the peptide sequence onto a support resin via a solid-phase coupling synthesis method to obtain the peptide-resin.

In the above manufacture method, the amount of the Fmoc-protected amino acid or protected amino acid fragment used is 1.2 to 6 equivalents relative to the total molar amount of the resin used, preferably 2.5 to 3.5 equivalents.

In the above manufacture method, the loading capacity of the solid support resin is 0.2 to 1.0 mmol/g resin, preferably 0.3 to 0.5 mmol/g resin.

As a preferred embodiment of the present disclosure, the solid-phase coupling synthesis method is carried out by removing the Fmoc protecting group from the protected amino acid-resin obtained in the previous step, followed by coupling with the next protected amino acid. The Fmoc deprotection is performed for 10 to 60 minutes, preferably 15 to 25 minutes. The coupling reaction is performed for 60 to 300 minutes, preferably 100 to 140 minutes.

The coupling reaction requires the addition of a coupling agent, which is selected from the group consisting of DIC (N,N-diisopropylcarbodiimide), N,N-dicyclohexylcarbodiimide, (benzotriazol- 1-yloxy)tripyrrolidinophosphonium hexafluorop ho sp hate, 2-(7-aza-1H-benzotriazol-1-yl)-1,1,3,3-tetramethyluronium hexafluorophosphate, benzotriazolyl-N,N,N',N'-tetramethyluronium hexafluorophosphate, and O-benzotriazolyl-N,N,N',N'-tetramethyluronium tetrafluoroborate; preferably N,N-diisopropylcarbodiimide. The molar amount of the coupling agent used is 1.2 to 6 equivalents relative to the total molar amount of amino groups in the amino-resin, preferably 2.5 to 3.5 equivalents.

The coupling reaction requires the addition of an activating agent, which is selected from the group consisting of 1-hydroxybenzotriazole and N-hydroxy-7-azabenzotriazole, preferably 1-hydroxybenzotriazole. The amount of the activating agent used is 1.2 to 6 equivalents relative to the total molar amount of amino groups in the amino-resin, preferably 2.5 to 3.5 equivalents.

As a preferred embodiment of the present disclosure, an agent for Fmoc deprotection is a mixed solution of PIP/DMF (piperidine/N,N-dimethylformamide), wherein the content of piperidine in the mixed solution is 10-30% (v/v). The amount of the Fmoc deprotection agent used is 5-15 mL per gram of amino-resin, preferably 8-12 mL per gram of amino-resin.

Preferably, the peptide-resin is subjected to acidolysis to simultaneously remove the resin and side-chain protecting groups to obtain a crude product.

Further preferably, an acidolysis agent used for acidolysis of the peptide-resin is a mixed solvent of trifluoroacetic acid (TFA), 1,2-ethanedithiol (EDT), and water, wherein the volume ratio of the mixed solvent is: 80-95% TFA, 1-10% EDT, and the balance being water.

More preferably, the volume ratio of the mixed solvent is: 89-91% TFA, 4-6% EDT, and the balance being water. Most preferably, the volume ratio of the mixed solvent is: 90% TFA, 5% EDT, and the balance being water.

The amount of the acidolysis agent used is 4-15 mL per gram of peptide-resin; preferably 7-10 mL per gram of peptide-resin.

The acidolysis using the acidolysis agent is performed for 1-6 hours at room temperature, preferably 3-4 hours.

Further, the crude product is purified by high-performance liquid chromatography and lyophilized to obtain the purified product.

### 1. Synthesis of peptide-resin

Rink amide MBHA resin was used as the solid support, and sequentially coupled with protected amino acids corresponding to the peptide sequence via Fmoc deprotection and coupling reactions to obtain a peptide-resin.

### (1) Coupling of the first protected amino acid of the main chain

0.03 mol of the first protected amino acid and 0.03 mol of HOBt were taken and dissolved in an appropriate amount of DMF. Separately, 0.03 mol of DIC was taken and slowly added into the solution of the protected amino acid in DMF under stirring. The mixture was stirred at room temperature for 30 minutes to obtain an activated solution of protected amino acid for later use.

0.01 mol of Rink amide MBHA resin (loading capacity: about 0.4 mmol/g) was taken and deprotected with 20% PIP/DMF solution for 25 minutes. The resin was washed and filtered to obtain Fmoc-deprotected resin.

The activated solution of the first protected amino acid was added to the Fmoc-deprotected resin, and a coupling reaction was performed for 60-300 minutes. The resin was then filtered and washed to obtain a resin bearing one protected amino acid.

### (2) Coupling of protected amino acids of main chain

The protected amino acids corresponding to the peptide sequence were sequentially coupled onto the resin by the same method as described for the first protected amino acid of the main chain, thereby obtaining a resin bearing the main chain amino acids.

### (3) Coupling of the first protected amino acid of the side chain

0.03 mol of the first protected amino acid of the side chain and 0.03 mol of HOBt were taken and dissolved in an appropriate amount of DMF. Separately, 0.03 mol of DIC was taken and slowly added into the solution of the protected amino acid in DMF under stirring. The mixture was stirred at room temperature for 30 minutes to obtain an activated solution of protected amino acid.

2.5 mmol of tetrakis(triphenylphosphine)palladium and 25 mmol of phenylsilane were taken and dissolved in an appropriate amount of dichloromethane. The resin was deprotected for 4 hours, then filtered and washed to obtain Alloc-deprotected resin for later use.

The activated solution of the first protected amino acid of the side chain was added to the Alloc-deprotected resin, and a coupling reaction was performed for 60-300 minutes. The resin was filtered and washed to obtain a resin bearing the first protected amino acid of the side chain.

### (4) Coupling of other protected amino acids or mono-protected fatty acids of side chain

The protected amino acids and mono-protected fatty acids corresponding to the side chain were sequentially coupled onto the resin by the same method as described for the first protected amino acid of the main chain, thereby obtaining the peptide-resin.

### 2. Manufacture of Crude Product

The above peptide-resin was taken, and a cleavage agent having a volume ratio of TFA:water:EDT = 95:5:5 (10 mL of cleavage agent per gram of resin) was added. The mixture was stirred uniformly and reacted at room temperature for 3 hours. The reaction mixture was filtered through a fritted funnel, and the filtrate was collected. The resin was further washed three times with a small amount of TFA, and the washings were combined with the filtrate. The combined solution was concentrated under reduced pressure, and anhydrous ether was added to precipitate the product. The precipitate was washed three times with anhydrous ether and dried under suction to obtain an off-white powder, which was the crude product.

### 3. Manufacture of purified products

The above crude product was taken, water was added with stirring, and the pH was adjusted to 8.0 with aqueous ammonia until the above crude product was completely dissolved. The solution was filtered through a 0.45 µm membrane and reserved for further use.

Purification was carried out by high-performance liquid chromatography. The chromatographic column packing used for purification was 10 µm reversed-phase C18, and the mobile phase system was 0.1% TFA in water - 0.1% TFA in acetonitrile. A chromatographic column (30 mm × 250 mm) was used at a flow rate of 20 mL/min. Gradient elution was employed, and cyclic injection and purification were performed. The crude product solution was loaded onto the chromatographic column, elution with the mobile phase was initiated, and the main peak was collected. After acetonitrile in the main peak was removed, a purified intermediate concentrate was obtained.

The purified intermediate concentrate was filtered through a 0.45 µm membrane and reserved for use. Desalting was then carried out by high-performance liquid chromatography. The mobile phase system was 1% acetic acid in water - acetonitrile, and the chromatographic column packing was 10 µm reversed-phase C18. A chromatographic column (30 mm × 250 mm) was used at a flow rate of 20 mL/min (the flow rate was adjusted accordingly depending on the column specifications). Gradient elution and cyclic loading were employed. The sample was loaded onto the chromatographic column, elution with mobile phase was initiated, and chromatograms were recorded while monitoring absorbance changes. The main desalting peak was collected and analyzed for purity by analytical liquid chromatography. The collected main peak fractions were combined and concentrated under reduced pressure to obtain an aqueous acetic acid solution of the purified product, which was then lyophilized to obtain the purified peptide.

The following compounds were synthesized according to the above methods:

| **Compound** | **Sequence number** | **Sequence structure** |
|---|---|---|
| Positive | SEQ ID | Tyr-Aib-Gln-Gly- Thr-Phe-Thr-Ser-Asp-Tyr-Ser-Ile-αMeLeu- |
| control; Retatrutide | No.1 | Leu-Asp-Lys-Lys(AEEA-γGlu-eicosanedioic acid)-Ala-Gln-Aib-Ala-Phe-Ile-Glu-Tyr-Leu-Leu-Glu-Gly-Gl y-Pro-Ser-Ser-Gly-Ala-Pro-Pro-Pro-Ser-NH2 |
| Compound 1 | SEQ ID No.2 | Tyr-Aib-Gln-Gly- Thr-αMePhe-Thr-Ser-Asp-Tyr-Ser-Ile-αMe Leu-Leu-Asp-Lys-Lys(AEEA-γGlu-eicosanedioic acid)-Ala-Gln-Aib-Ala-Phe-Ile-Glu-Tyr-Leu-Leu-Glu-(5-Ava) -Pro-Ser-Ser-Gly-Ala-Pro-Pro-Pro-Ser-NH2 |
| Compound 2 | SEQ ID No.3 | Tyr-Aib-Gln-Gly-Thr-αMePhe-Thr-Ser-Asp-Tyr-Ser-Ile-αMe Leu-Leu-Asp-Lys-Lys(AEEA-δAad-eicosanedioic acid)-Ala-Gln-Aib-Ala-Phe-Ile-Glu-Tyr-Leu-Leu-Glu-(5-Ava) -Pro-Ser-Ser-Gly-Ala-Pro-Pro-Pro-Ser-NH2 |
| Compound 3 | SEQ ID No.4 | Tyr-Aib-Gln-Gly- Thr-αMePhe-Thr-Ser-Asp-Tyr-Ser-Ile-αMe Leu-Leu-Asp-Lys-Lys(AEEA-εApm-eicosanedioic acid)-Ala-Gln-Aib-Ala-Phe-Ile-Glu-Tyr-Leu-Leu-Glu-(5-Ava) -Pro-Ser-Ser-Gly-Ala-Pro-Pro-Pro-Ser-NH2 |
| Compound 4 | SEQ ID No.5 | Tyr-Aib-Gln-Gly- Thr-αMePhe-Thr-Ser-Asp-Tyr-Ser-Ile-αMe Leu-Leu-Asp-Lys-Lys(AEEA-ζAsu-eicosanedioic acid)-Ala-Gln-Aib-Ala-Phe-Ile-Glu-Tyr-Leu-Leu-Glu-(5-Ava) -Pro-Ser-Ser-Gly-Ala-Pro-Pro-Pro-Ser-NH2 |
| Compound 5 | SEQ ID No.6 | Tyr-Aib-Gln-Gly- Thr-αMePhe(2F)- Thr-Ser-Asp-Tyr-Ser-Ile-α MeLeu-Leu-Asp-Lys-Lys(AEEA-γGlu-eicosanedioic acid)-Ala-Gln-Aib-Ala-Phe-Ile-Glu-Tyr-Leu-Leu-Glu-(5-Ava) -Pro-Ser-Ser-Gly-Ala-Pro-Pro-Pro-Ser-NH2 |
| Compound 6 | SEQ ID No.7 | Tyr-Aib-Gln-Gly- Thr-αMePhe(2F)- Thr-Ser-Asp-Tyr-Ser-Ile-α MeLeu-Leu-Asp-Lys-Lys(AEEA-δAad-eicosanedioic acid)-Ala-Gln-Aib-Ala-Phe-Ile-Glu-Tyr-Leu-Leu-Glu-(5-Ava) -Pro-Ser-Ser-Gly-Ala-Pro-Pro-Pro-Ser-NH2 |
| Compound 7 | SEQ ID No.8 | Tyr-Aib-Gln-Gly- Thr-αMePhe(2F)- Thr-Ser-Asp-Tyr-Ser-Ile-α MeLeu-Leu-Asp-Lys-Lys(AEEA-εApm-eicosanedioic acid)-Ala-Gln-Aib-Ala-Phe-Ile-Glu-Tyr-Leu-Leu-Glu-(5-Ava) -Pro-Ser-Ser-Gly-Ala-Pro-Pro-Pro-Ser-NH2 |
| Compound 8 | SEQ ID No.9 | Tyr-Aib-Gln-Gly- Thr-αMePhe(2F)- Thr-Ser-Asp-Tyr-Ser-Ile-α MeLeu-Leu-Asp-Lys-Lys(AEEA-ζAsu-eicosanedioic acid)-Ala-Gln-Aib-Ala-Phe-Ile-Glu-Tyr-Leu-Leu-Glu-(5-Ava) -Pro-Ser-Ser-Gly-Ala-Pro-Pro-Pro-Ser-NH2 |
| Compound 9 | SEQ ID No.10 | Tyr-Aib-Gln-Gly- Thr-αMePhe-Thr-Ser-Asp-Tyr-Ser-Ile-αMe Leu-Leu-Asp-Lys-Lys(AEEA-AEEA-γGlu-eicosanedioic acid)-Ala-Gln-Aib-Ala-Phe-Ile-Glu-Tyr-Leu-Leu-Glu-(5-Ava) -Pro-Ser-Ser-Gly-Ala-Pro-Pro-Pro-Ser-NH2 |
| Compound 10 | SEQ ID No.11 | Tyr-Aib-Gln-Gly- Thr-αMePhe-Thr-Ser-Asp-Tyr-Ser-Ile-αMe Leu-Leu-Asp-Lys-Lys(COCH2PEG5-γGlu-eicosanedioic acid)-Ala-Gln-Aib-Ala-Phe-Ile-Glu-Tyr-Leu-Leu-Glu-(5-Ava) -Pro-Ser-Ser-Gly-Ala-Pro-Pro-Pro-Ser-NH2 |
| Compound 11 | SEQ ID No.12 | Tyr-Aib-Gln-Gly- Thr-αMePhe(2F)- Thr-Ser-Asp-Tyr-Ser-Ile-α MeLeu-Leu-Asp-Lys-Lys(AEEA-AEEA-γGlu-eicosanedioic acid)-Ala-Gln-Aib-Ala-Phe-Ile-Glu-Tyr-Leu-Leu-Glu-(5-Ava) -Pro-Ser-Ser-Gly-Ala-Pro-Pro-Pro-Ser-NH2 |
| Compound 12 | SEQ ID No.13 | Tyr-Aib-Gln-Gly- Thr-αMePhe(2F)- Thr-Ser-Asp-Tyr-Ser-Ile-α MeLeu-Leu-Asp-Lys-Lys(COCH2PEG5-γGlu-eicosanedioic acid)-Ala-Gln-Aib-Ala-Phe-Ile-Glu-Tyr-Leu-Leu-Glu-(5-Ava) -Pro-Ser-Ser-Gly-Ala-Pro-Pro-Pro-Ser-NH2 |

### Example 2: Activity test

### 1. GLP-1 activity test method

Upon stimulation by a specific agonist, GLP-1R activates the intracellular adenylate cyclase signaling pathway, resulting in an increase in cAMP levels and ultimately leading to insulin production and secretion. A cell line stably transfected with GLP-1R was stimulated with test compounds, thereby rapidly increasing intracellular cAMP levels. The relative luminescence units (RLU) of cells stimulated at various concentrations were measured by a chemiluminescence method, and the EC50 value of the agonist was calculated accordingly. This activity test method is currently a commonly used method for evaluating GLP-1 receptor agonist activity both domestically and internationally.

A CHO-K1 cell line stably expressing GLP-1R was used. The stable cells were stimulated with agonists at different concentrations, and the relative luminescence units after stimulation at each concentration were measured to calculate the EC₅₀ value of the agonist.

### 2. GIP activity test method

Upon stimulation by a specific agonist, GIPR activates the intracellular adenylate cyclase signaling pathway, resulting in an increase in cAMP levels and ultimately leading to insulin production and secretion. A cell line stably transfected with GIPR was stimulated with test compounds, thereby rapidly increasing intracellular cAMP levels. The relative luminescence units (RLU) of cells stimulated at various concentrations were measured by a chemiluminescence method, and the EC50 value of the agonist was calculated accordingly. This activity assay method is currently a commonly used method for evaluating GIP receptor agonist activity both domestically and internationally.

A CHO-K1 cell line stably expressing GIPR was used. The stable cells were stimulated with agonists at different concentrations, and the relative luminescence units after stimulation at each concentration were measured to calculate the EC₅₀ value of the agonist.

### 3. GCG activity test method

Upon stimulation by a specific agonist, GCG-R activates the intracellular adenylate cyclase signaling pathway, resulting in an increase in cAMP levels and ultimately leading to insulin production and secretion. A cell line stably transfected with GCG-R was stimulated with test compounds, thereby rapidly increasing intracellular cAMP levels. The relative luminescence units (RLU) of cells stimulated at various concentrations were measured by a chemiluminescence method, and the EC50 value of the agonist was calculated accordingly. This activity assay method is currently a commonly used method for evaluating GCG receptor agonist activity both domestically and internationally.

A CHO-K1 cell line stably expressing GCG-R was used. The stable cells were stimulated with agonists at different concentrations, and the relative luminescence units after stimulation at each concentration were measured to calculate the EC₅₀ value of the agonist.

### 4. Result of the test

The result of the test is shown in the following table:

| **Compound** | **GLP-1 [EC₅₀(pmol)]** | **GIP [EC₅₀(pmol)]** | **GCG [EC₅₀(pmol)]** |
|---|---|---|---|
| Positive control; retatrutide | 46.0 | 34.2 | 523.5 |
| Compound 1 | 45.0 | 24.4 | 271.4 |
| Compound 2 | 40.6 | 18.1 | 243.7 |
| Compound 3 | 45.2 | 26.7 | 283.0 |
| Compound 4 | 55.4 | 31.0 | 286.1 |
| Compound 5 | 43.4 | 23.6 | 158.5 |
| Compound 6 | 37.5 | 18.8 | 141.0 |
| Compound 7 | 45.5 | 24.6 | 162.6 |
| Compound 8 | 52.4 | 32.9 | 179.2 |
| Compound 9 | 51.9 | 26.8 | 307.5 |
| Compound 10 | 49.4 | 25.6 | 299.3 |
| Compound 11 | 48.7 | 25.3 | 173.5 |
| Compound 12 | 46.9 | 24.3 | 165.3 |

Experimental results show that, while maintaining the activity of GLP-1, the activity of GIP of the compounds of examples was increased to varying degrees, while the activity of GCG was significantly increased.

### Example 3: Preliminary pharmacokinetic characterization evaluation

Among the above compounds, the principle of modification for long-acting was essentially the same. Therefore, the two compounds exhibiting the best activity were selected for preliminary pharmacokinetic evaluation.

The test animals were male cynomolgus monkeys. Two animals were used for each compound. The compounds were administered subcutaneously at a dose of 0.2 mg/kg. Blood samples were collected via intravenous sampling prior to administration (0 h) and at 1 h, 2 h, 3 h, 4 h, 8 h, 12 h, 18 h, 24 h, 48 h, 96 h, 144 h, and 168 h after administration. Plasma samples were obtained by centrifugation. The plasma concentrations of the compounds were respectively determined by liquid chromatography - mass spectrometry (LC-MS). The half-lives of the compounds administered subcutaneously (SC) are shown in the table below:

| **Compound** | **t_{1/2} (h)** |
|---|---|
| Compound 2 | 107.6 |
| Compound 6 | 115.2 |

The preliminary pharmacokinetic results in cynomolgus monkeys showed that the half-lives of Compound 2 and Compound 6 administered subcutaneously were both greater than 100 hours, fully meeting the requirements for long-acting administration.

The tri-agonist compounds provided by the present disclosure are described in detail above. The principles and implementation of the present disclosure are illustrated by specific examples. The description of the above examples is intended only to facilitate understanding of the methods and core concepts of the present disclosure. It should be noted that, for those skilled in the art, various improvements and modifications can be made without departing from the principles of the present disclosure, and such improvements and modifications shall fall within the scope of the claims of the present disclosure.

## Claims

1. A GLP-1/GIP/GCG tri-agonist compound having a structure of Formula I: 
Tyr-Aib-Gln-Gly-Thr-AA1-Thr-Ser-Asp-Tyr-Ser-Ile-αMeLeu-Leu-Asp-Lys-Lys((CO(CH2)n1PEG
n2)n3-(AA2)n4-CO(CH2)n5-COOH)-Ala-Gln-Aib-Ala-Phe-Ile-Glu-Tyr-Leu-Leu-Glu-(5-Ava)-Pro-S
er-Ser-Gly-Ala-Pro-Pro-Pro-Ser-AA3

Formula I
 wherein:
AA1 is any one selected from the group consisting of αMePhe and αMePhe(2F);
AA2 is any one selected from the group consisting of γGlu, δAad, εApm, and ζAsu;
AA3 is any one selected from the group consisting of amino and hydroxyl;
n1=1-5, n2=1-30, n3=0 or n3=1-5, n4=0 or n4=1-5, and n5=10-30.

2. The GLP-1/GIP/GCG tri-agonist compound according to claim 1, wherein AA1 is αMePhe, AA2 is γGlu, AA3 is amino, n1=1-5, n2 =1-30, n3=0 or n3 =1-5, n4=0 or n4=1-5, and n5=10-30.

3. The GLP-1/GIP/GCG tri-agonist compound according to claim 2, wherein AA1 is αMePhe, AA2 is γGlu, AA3 is amino, n1=1, n2=2, n3=1, n4=1, and n5=18.

4. The GLP-1/GIP/GCG tri-agonist compound according to claim 1, wherein AA1 is αMePhe, AA2 is δAad, AA3 is amino, n1=1-5, n2=1-30, n3=0 or n3=1-5, n4=0 or n4=1-5, and n5=10-30.

5. The GLP-1/GIP/GCG tri-agonist compound according to claim 4, wherein AA1 is αMePhe, AA2 is δAad, AA3 is amino, n1=1, n2=2, n3=1, n4=1, and n5=18.

6. The GLP-1/GIP/GCG tri-agonist compound according to claim 1, wherein AA1 is αMePhe(2F), AA2 is γGlu, AA3 is amino, n1=1-5, n2=1-30, n3=0 or n3=1-5, n4=0 or n4=1-5, and n5=10-30.

7. The GLP-1/GIP/GCG tri-agonist compound according to claim 6, wherein AA1 is αMePhe(2F), AA2 is γGlu, AA3 is amino, n1=1, n2=2, n3=1, n4=1, and n5=18.

8. The GLP-1/GIP/GCG tri-agonist compound according to claim 1, wherein AA1 is αMePhe(2F), AA2 is δAad, AA3 is amino, n1=1-5, n2=1-30, n3=0 or n3=1-5, n4=0 or n4=1-5, and n5=10-30.

9. The GLP-1/GIP/GCG tri-agonist compound according to claim 8, wherein AA1 is αMePhe(2F), AA2 is δAad, AA3 is amino, n1=1, n2=2, n3=1, n4=1, and n5=18.

10. The GLP-1/GIP/GCG tri-agonist compound according to any one of claims 1 to 9, wherein the compound has:
(I) an amino acid sequence set forth in any one of SEQ ID NOs: 2 to 13;
(II) an amino acid sequence obtained by substitution, deletion, or addition of 1, 2, 3, or more amino acids to the amino acid sequence of (I) and having the same function as the amino acid sequence of (I); or
(III) an amino acid sequence having 60%, 65%, 70%, 75%, 80%, 85%, 90%, 95%, 96%, 97%, 98%, 99% or more sequence identity to the amino acid sequence of (I) or (II).

11. The GLP-1/GIP/GCG tri-agonist compound according to any one of claims 1 to 10, wherein the tri-agonist compound includes a pharmaceutically acceptable salt, chelate, or non-covalent complex formed by the compound, a precursor of the compound, or any mixture thereof.

12. Use of the GLP-1/GIP/GCG tri-agonist compound according to any one of claims 1 to 11 in the manufacture of a pharmaceutical composition for treating a disease.

13. The use according to claim 12, wherein the pharmaceutical composition is for treating at least one disease selected from the group consisting of type II diabetes, impaired glucose tolerance, type I diabetes, obesity, hypertension, metabolic syndrome, dyslipidemia, cognitive disorder, atherosclerosis, myocardial infarction, coronary heart disease, cardiovascular disease, stroke, inflammatory bowel syndrome and/or dyspepsia or gastric ulcer, hepatic fibrosis disease, and pulmonary fibrosis disease.

14. A drug, comprising the GLP-1/GIP/GCG tri-agonist compound according to any one of claims 1 to 11.

15. A pharmaceutical composition, comprising the GLP-1/GIP/GCG tri-agonist compound according to any one of claims 1 to 11 and any additional active ingredient.

16. A method for treating a disease, comprising administering the GLP-1/GIP/GCG tri-agonist compound according to any one of claims 1 to 11, the drug according to claim 14, or the pharmaceutical composition according to claim 15.
